# EUROPEAN PATENT SPECIFICATION

(11) **EP 2 954 918 B1**
(45) Date of publication and mention of the grant of the patent: **30.09.2020**
(21) Application number: 14305878.2
(22) Date of filing: 11.06.2014
(51) Int. Cl.: A61M 16/06, A61M 39/10

(54) **New structure for a nasal respiratory mask**
Neue Struktur für eine nasale Atemmaske
Nouvelle structure pour un masque respiratoire nasal

(43) Date of publication of application: 16.12.2015
(73) Proprietor: Air Liquide Medical Systems, 92160 Antony (FR)
(72) Inventor: Masserdotti, Fulvio, 25075 Brescia (IT); Alberici, Luca, 25128 Brescia (IT); Sandoni, Giuseppe, 25124 Brescia (IT)
(74) Representative: Pittis, Olivier

(56) References cited:
- EP-A2- 2 005 986
- US-A1- 2009 223 521
- US-A1- 2012 234 326
- US-A1- 2013 139 822
- US-A1- 2014 083 430

## Description

The invention concerns a respiratory mask, in particular a nasal mask, in particular for adult or pediatric uses in the treatment of respiratory conditions or diseases affecting adults, children, toddlers, infants or newborns.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD).

Nasal masks deliver a flow of breathable gas for or to assist in patient respiration.

Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face. This face-contacting cushion is usually made of soft, resilient elastomeric material, such as soft silicone or similar material. Mask additionally may have a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face, which holding arm is also usually made of polymer. The hollow shell typically receives a gas supply line which delivers a respiratory gas, such as air under pressure, into the breathing chamber of the shell. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by EP-A-462701, EP-A-874667, EP-A-1972357 or WO-A-00/57942. Respiratory masks are disclosed also in EP2005986A2, US2009/223521 A1, US2013/139822A1, US2012/234326A1 and US2014/083430A1.

However, the current masks have often a complicated architecture resulting in masks that are often heavy to wear and cumbersome for the patients, especially when patients have to wear these masks overnight.

This leads also to some difficulties for well-positioning the mask on the face of said patients and maintaining it thereafter, without gas leaks and discomfort for the patients.

Due to their current conceptions, the size and weight of a lot of existing masks, such as nasal masks, are excessive and hence should be improved for the benefit of the patients, especially for avoiding or minimizing the above mentioned issues and discomforts.

Hence, the problem to be solved is to provide an improved respiratory mask architecture, especially a nasal mask, that is light and comfortable to wear, thanks to reduced size and weight, as well as a simplified architecture, thereby ensuring efficient positioning and securing of the mask on the patient's face, a good tightness (seal) preventing the escape of gas and improved comfort of use for the patient.

The solution of the present invention concerns a respiratory mask, in particular a nasal mask, constituted of 4 parts assembled together comprising :
- a first part made of a main body having a tubular-shape portion and a central passage, a holding arm projecting upwardly from said main body, and two lateral arms projecting laterally from said main body, the two lateral arms and the holding arm being integrally fixed to said tubular-shape portion of the main body,
- a second part comprising a flexible cushion fixed to the rear side of the tubular-shape portion of the main body,
- a third part comprising a hollow curved connector fixed to the front side of the tubular-shape portion of the main body, said hollow curved connector being rotatable with respect to the main body, and
- a fourth part comprising a tubular connecting piece detachably fixed to the hollow curved connector, said tubular connecting piece being rotatable with respect to the hollow curved connector.

A mask according to the present invention has a very simple structure made of only 4 parts or sub-units assembled together, resulting in a notable reduction of size and weight. The invention is defined by the appended claim 1.

The mask according to the present disclosure can further comprise one or more of the following additional features:
- the main body is essentially formed by the tubular-shape portion.
- the main body is constituted by a ring- or tube- element or piece forming the tubular-shape portion.
- the tubular-shape portion has a generally cylindrical form, tronconical form or a combination thereof.
- the two lateral arms and the holding arm are integral with the peripheral wall of said tubular portion.
- the tubular-shape portion forming the main body, the holding arm and of the two lateral arms are molded in one piece.
- the tubular-shape portion forming the main body, the holding arm and of the two lateral arms are made of a flexible material,
- the tubular-shape portion forming the main body, the holding arm and of the two lateral arms are made of a polymer material, such as polycarbonate (PC), polypropylene (PP) or nylon.
- the two lateral arms and the holding arm each comprises a proximal end integral with the tubular-shape portion of the main body, and a free distal end.
- the tubular portion is traversed by a central passage for conveying a gas, said central passage comprising an inlet orifice and an outlet orifice having each a diameter of between 1 and 2.5 cm.
- the tubular-shape portion of the main body has a length of between 0.5 and 3cm.
- the holding arm and of the two lateral arms have an elongated shape.
- the holding arm and of the two lateral arms have for example a band or ribbon shape. Other shapes are possible.
- the holding arm has a length of about 2 and 8 cm.
- each lateral arms has a length of about 3 and 6 cm.
- the free distal ends of the holding arm and of the two lateral arms comprise a strap-fixing system for fixing thereto the straps of a headgear.
- the main body, the holding arm and of the two lateral arms are made of a flexible polymeric material so as to be slightly bendable toward the patient's face under the forces applied by the headgear, when said headgear is adjusted to the morphology of the patient's head.
- the strap-fixing system carried by the free distal ends of the holding arm and of the two lateral arms comprise at least one slot and/or fixing hook for fixing straps of a headgear.
- the strap-fixing system carried by the free distal end of the holding arm comprises at least one slot, preferably two slots, such as open slots. For instance, the slots can have a width of between 8 to 35 mm.
- the strap-fixing system carried by each free distal ends of the two lateral arms comprises a fixing hook.
- the flexible cushion comprises a respiratory chamber, i.e. an inner chamber, with an aperture (i.e. a nose aperture) adapted for receiving at least part of the patient's nose, when the patient wears the mask, and an inlet orifice, said respiratory chamber being in fluid communication with the central passage of the tubular portion of the main body through said inlet orifice.
- the flexible cushion further comprises one or several membranes, preferably two superimposed membranes that ensure an efficient gas tightness while being in contact with the patient's face, when the latter wears the mask.
- the flexible cushion further comprises one or several membranes arranged around along or part of the periphery of the aperture of the respiratory chamber. In other words, the membrane(s) form(s) a flexible skirt around the along or part of the periphery of the aperture of the respiratory chamber.
- at least a region of the holding arm and of the two lateral arms is configured or shaped so as to match, the external profile, i.e. the outer contour, of the flexible cushion.
- the holding arm and the two lateral arms are configured to have a curved-shape.
- the main body further comprises a hollow curved connector fixed to the front side of the tubular-shape portion of the main body so as to be in fluid communication with one another thereby allowing a gas to circulate from the hollow curved connector to the central passage of the tubular-shape portion of the main body, or vice versa.
- the hollow curved connector is fixed to and rotatable with respect to the tubular-shape portion of the main body.
- the hollow curved connector has general "L" shape.
- the hollow curved connector comprises (one or several) lip portions cooperating with a groove arranged into the tubular connecting piece for ensuring a rotatable and detachable connection of the tubular connecting piece to the hollow curved connector.
- the upstream end of the tubular connecting piece is configured for connecting a gas line thereto.
- the main body further comprises a headgear comprising several straps connected to the free distal ends of the holding arm and of the two lateral arms. Said headgear is used for maintaining and securing the mask in a desired position on the head of the patient.
- the main body further comprises a headgear comprising several straps made of polymer material or fabric material, or both.
- the flexible cushion comprises first connecting structures arranged around its inlet orifice, and the rear side of the tubular-shape portion of the main body comprises second connecting structures, said first connecting structures cooperating with said second connecting structures so as to fix and maintain said flexible cushion integral with the main body.
- said first connecting structures comprise male/female connection elements, such as grooves, walls, abutments.
- the cushion is made of a soft flexible material.
- the cushion is made of silicone or similar.
- the cushion comprises an internal chamber and an inlet orifice in fluid communication with the internal chamber.
- the membrane and the cushion are integral and formed by a single piece made of a resilient soft or semi-soft material, i.e. of silicone or similar.
- the cushion has a general triangular, trapezoidal or saddle shape.
- the inlet orifice is arranged at the center of the cushion.
- the mask is a nasal mask.
- the cushion is configured and sized for coming into contact, when the mask is worn by the user or patient, with specific regions of the user or patient's face. Preferably, those regions comprise an intermediate nose region, an upper lip region and lateral nose regions, wherein
   - the upper lip region is the area comprised between the nose and the upper lip;
   - the intermediate nose region is the area of the nose approximately located at the junction of bone and cartilage; and
   - the lateral nose regions are those located on each side of the nose.

The invention also concerns an assembly comprising a gas delivery device, such as a medical ventilator, and a respiratory mask according to the present invention, preferably the gas delivery device is connected to the respiratory mask by means of a gas line, such as a flexible hose, connected to the tubular connecting piece fixed to the hollow curved connector, said hollow curved connector being itself fixed to the front side of the tubular portion of the main body of the mask.

An embodiment of a respiratory mask, especially a nasal mask, according to the present invention is shown in the enclosed Figures, among which:
- Figure 1 represents the main parts of a mask according to the present invention,
- Figure 2 is a rear view of the mask of Figure 1,
- Figure 3 is a sectional view of the mask of Figures 1 and 2,
- Figure 4 is an enlarged view of a part of Figure 3,
- Figure 5 is an enlarged view of the main body of the mask of Figures 1 to 3,
- Figures 6-8 show the mask of Figure 1 worn by a patient, and
- Figure 9 shows the tubular connecting piece and the hollow curved connector.

The present invention proposes a new structure of respiratory mask as shown in Figures that illustrate one embodiment of a nasal mask according to the present invention.

Generally speaking, a respiratory mask according to the present invention is constituted of 4 parts assembled together.

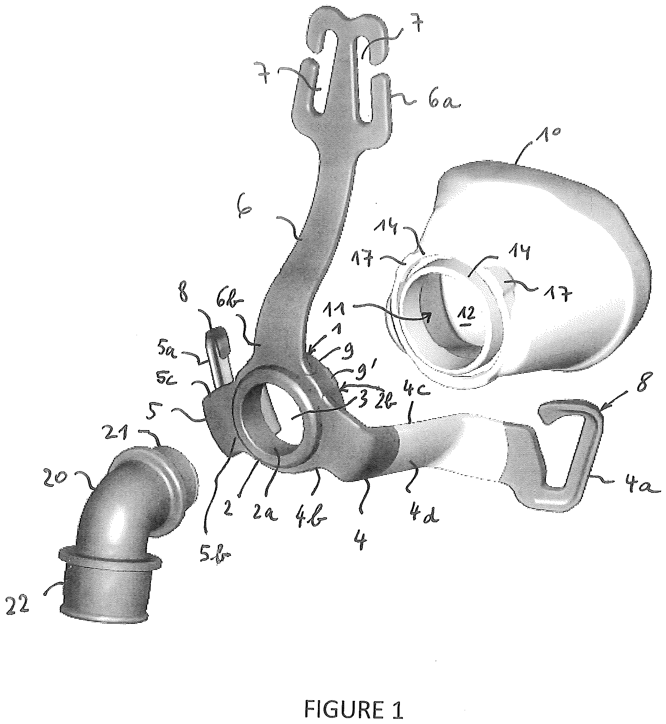

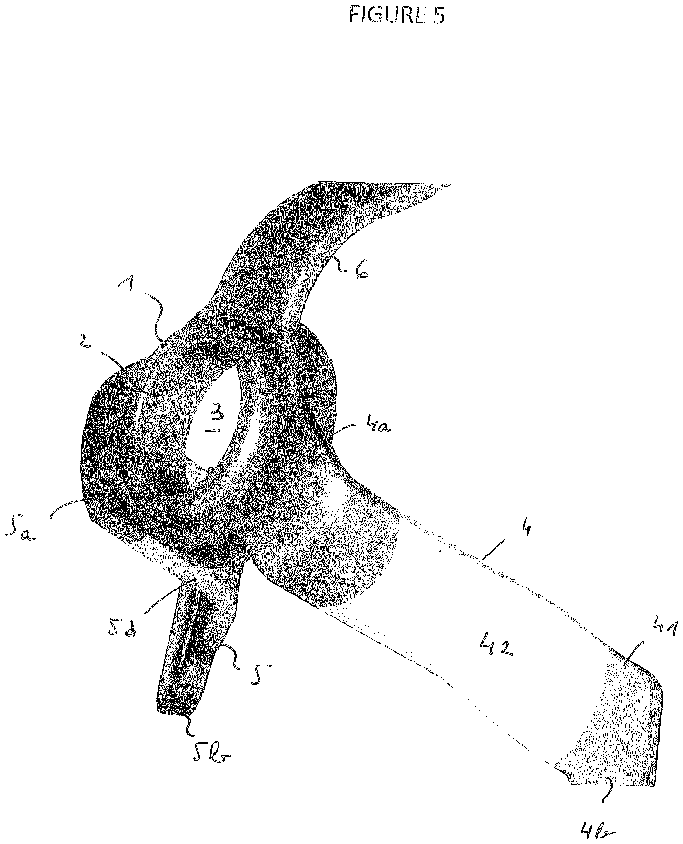

The first part of the mask comprises a mask body 1 having a particular simple structure as it is formed of a tubular-shape portion 2 traversed by a central passage 3as visible in Figures 1 and 5. In other words, the main body 1 of the mask according to the invention is essentially constituted of a hollow ring or tube element forming the tubular-shape portion 2 and the central passage 3.

The tubular-shape portion 2 is further carrying two lateral arms 4, 5 and a holding arm 6 that are integrally fixed to the peripheral wall or surface of said tubular-shape portion 2.

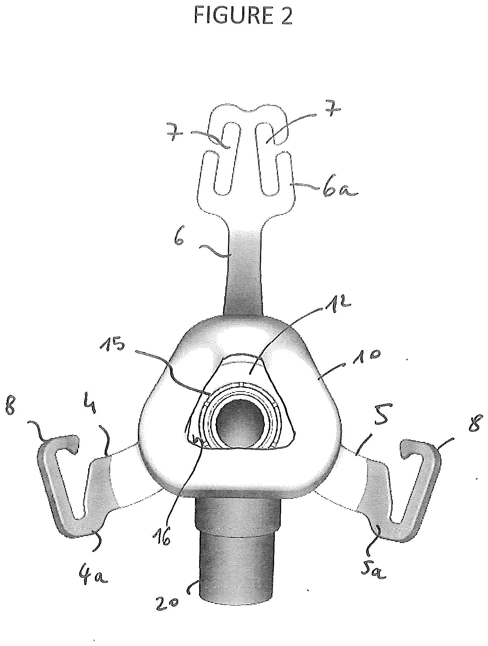

A holding arm 6 projects upwardly, i.e. about vertically, from said main body 2, whereas two lateral arms 4, 5 projecting laterally from said main body 1 in opposite directions, i.e. one toward the right side of the mask and the other toward the left side of the mask as shown in Figures 1 and 2.

Preferably, the tubular-shape portion 2, the two lateral arms 4, 5 and the holding arm 6 are molded in one piece. Nevertheless, they can be made from several pieces or sub-units that are subsequently assembled together and made integral by means of any available fixing technique, for instance they can be glued, thermo-welded or similarly affixed to one another.

For example, the tubular-shape portion 2, the two lateral arms 4, 5 and the holding arm 6 are made of a first material that is slightly flexible, preferably a polymer or plastic material, such as polypropylene (PP), polycarbonate (PC) and nylon, or similar.

Further, in order to increase the flexibility of the two lateral arms 4, 5, it is provided on each lateral arm 4, 5, an intermediary zone 4d, 5d exhibiting a flexibility that is higher than the flexibility of the rest of each of the two lateral arms 4, 5. In this aim, said intermediary zones 4d, 5d can be made of or comprise a second flexible material that exhibits a flexibility that is higher than the flexibility of the first material forming the rest of the two lateral arms 4, 5. For example, the second flexible material can be silicone or similar, whereas the first material can be PC, PP or nylon. The intermediary zones 4d, 5d of the two lateral arms 4, 5 are located between their distal 4a, 5a, and their proximal ends 4b, 5b, as shown in Figures 1 and 5.

According to the present invention, the tubular-shape portion 2 forming the main body of the mask is made of a ring-shape or tube-shape element or piece that forms the main body of the mask 1 or at least a predominant part of it.

That is to say that the tubular-shape portion 2 has a generally cylindrical or tronconical form or shape, or any combinations thereof. For example, the tubular-shape portion 2 forming the main body can be partially cylindrical and partially tronconical, or generally cylindrical but formed of sub-portions having different diameters.

Preferably, the tubular-shape portion 2 has a length of between 1.5 and 8 cm and its central passage 3 has a diameter of between 1 and 4 cm.

Further, the two lateral arms 4, 5 and the holding arm 6 have preferably respective lengths of between 2 and 15 cm.

Furthermore, the two lateral arms 4, 5 and the holding arm 6 are radially arranged and spaced on the tubular portion 2 of the main body 1 of the mask. Preferably, the vertical axis of the holding arm 6 forms an angle of between 90 and 150° with the axis of each of the lateral arms 4, 5.

The two lateral arms 4, 5 and the holding arm 6 that are integrally attached to the peripheral wall or surface of said tubular-shape portion 2 by means of their proximal ends 4b, 5b, 6b. They further each comprise a free distal end 4a, 5a, 6a comprising a strap-fixing system 7, 8 for fixing thereto the straps of a headgear that is used for maintaining and securing the mask in a desired position on the head of the patient. Generally, the straps of headgears are made of polymer or fabric materials.

In the present case, the strap-fixing system 7, 8 carried by the free distal end 6a of the holding arm 6 comprises an enlarged structure comprising two open slots 7, whereas the free distal ends 4a, 5a of the two lateral arms 4, 5 each comprise a fixing hook 8 for fixing headgear straps forming loops.

Further, the mask of the present invention also comprises a second part formed by a flexible cushion 2 that is fixed to the rear side 2b of the tubular-shape portion 2 of the main body 1

Typically, the flexible cushion 10 is a tridimensional hollow structure forming a respiratory chamber 12 with a nose aperture 15 adapted, i.e. conformed and sized, for receiving at least part of the patient's nose as above explained, when the patient wears the mask, so that the patient can breathe the respiratory gas contained in said chamber 12.

The cushion body further comprises an inlet orifice 11 for allowing a gas or gas mixture, such as under pressure air, to enter into the respiratory chamber 12

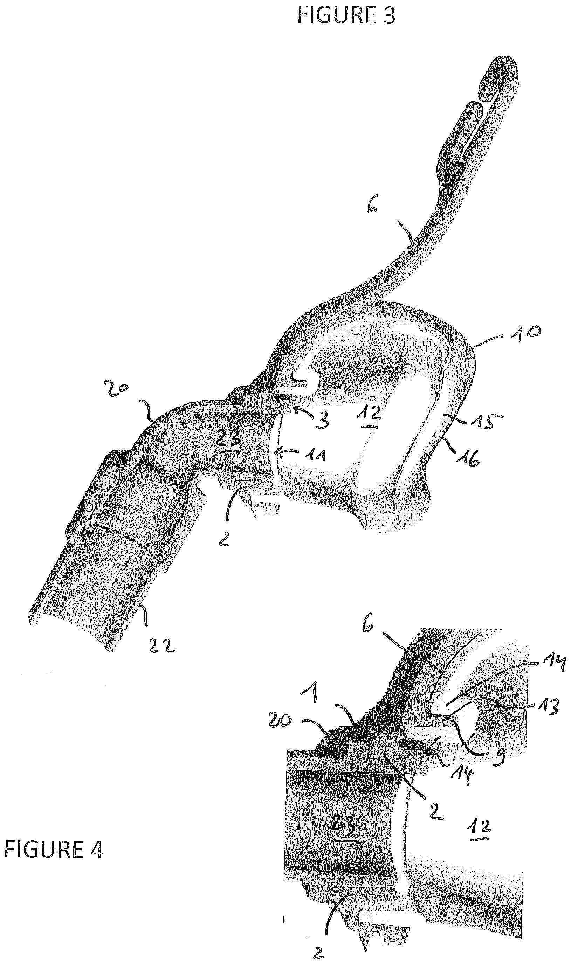

As shown in Figure 3, the respiratory chamber 12 is in fluid communication with the central passage 3 of the tubular-shape portion 2 of the main body 1 and/or with the lumen 23 of the hollow curved connector 20, through said inlet orifice 11 so that a gas flow can travel from said tubular connector 20 to said chamber 12.

Preferably, in order to provide efficient gas tightness (i.e. seal) and/or increased comfort for the patient, the nose aperture 15 of the cushion 10 is delimited by at least one flexible membrane 16 that comes into contact with the patient's face and matches his/her facial morphology.

In other words, the membrane(s) constitute(s) a kind of soft flexible skirt delimiting said the nose aperture 15 of the cushion 10. Preferably, two (or more) superimposed membranes 16 are used as using several membranes may improve the gas tightness.

As shown in Figure 2, the cushion 10 has, in the present case, a generally trapezoidal or saddle tridimensional form or shape (rear view) so as to better match the contours of the patient's face, especially in the nasal regions.

When the mask is worn by the patient, i.e. when the cushion 10 receives at least a part of the patient's nose, when said patient introduces his/her nose into the internal volume of the breathing chamber12 and breathes the gas contained therein, the cushion 10 and the membrane(s) 16 arranged around the peripheral border of the nose aperture 15 are in contact with the intermediate nose region (area at the junction of the bone and cartilage of the nose), the upper lip region (area between the nose and the upper lip), and the two opposite lateral regions of the nose of the patient (i.e. right and left flange regions of the nose). Of course, other embodiments and shapes are possible.

The cushion 10 is preferably made of a flexible and soft material, such as silicone or the like. Preferably, the membrane(s) 16 and the cushion body are molded in one piece.

The cushion 10 is fixed to the tubular-shape portion 2 of the main body 1 by means of a specific connecting system, such as male/female connections.

In the present embodiment, as illustrated in Figures 3 and 4, the flexible cushion 10 comprises first connecting structures 13, 14, 17 cooperating with second connecting structures 9, 9' arranged on the rear side 2b of the tubular-shape portion 2 of the main body 2 so as to fix and maintain said flexible cushion 10 integral with the main body 1 and to ensure a gas tightness in-between.

For example, the first connecting structures 13, 14, 17 can comprise first peripheral groove(s) 13 and wall(s) 14, as arranged around the inlet orifice 11 of the cushion 10, whereas the second connecting structures 9, 9' can comprise second peripheral wall(s) 9 and/or groove(s) that are configured or shaped so as to perfectly fit together.

The first connecting structures 13, 14, 17 of the cushion 10 can also comprise one or several abutments 17 cooperating with corresponding lodgings 9' of the second connecting structures 9, 9' carried by the main body 2, while those abutments 17 are inserted into said lodgings 9'. Such couples of abutments 17/lodgings 9' can be used for ensuring a suitable alignment of the cushion 10 vis-a-vis the main body 2.

Moreover, as shown in Figure 1 and 3, the two lateral arms 4, 5 and the holding arm 6 of the main body 2 have elongated structures, such as band or ribbon -like forms, and are slightly incurved so as to match the contours of the cushion 10. In other words, some regions 6c, 4c, 5c of the holding arm 6 and of the two lateral arms 4, 5 are configured or shaped so as to conform to the external profile of the cushion 10.

As shown in Figure 1, as already mentioned, a hollow curved connector 20 is fixed to the front side 2a of the tubular-shape portion 2 of the main body 1 so as to be in fluid communication with one another.

Said hollow curved connector 20 has a general bent tubular-shape, namely a kind of "L"-shape and comprises a lumen 23 or inner passage for conveying gas under pressure to the mask body 2 and cushion 10.

A respiratory gas, such as pressurized air, is introduced into the lumen 23 of the hollow curved connector 20 forming a third part of the mask, to which is connected a gas feeding line, such as a flexible hose, by means of a tubular connecting piece 22 that has a tubular shape.

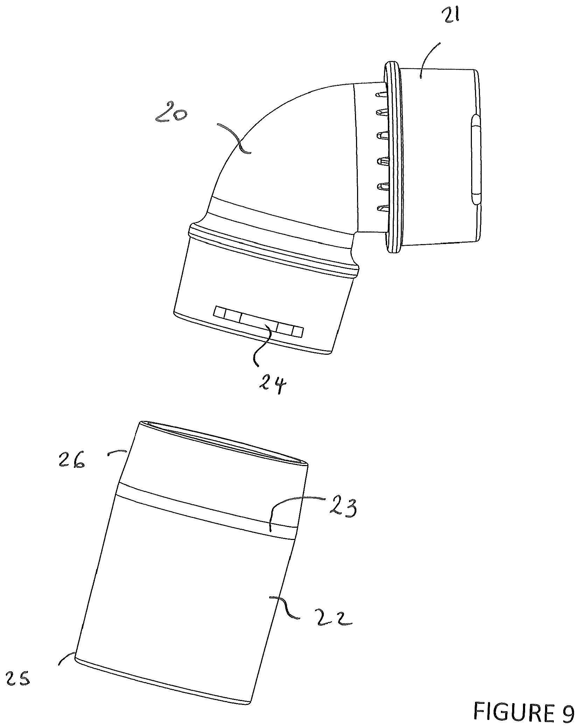

The tubular connecting piece 22 that forms the fourth part of the mask of the invention is used for connecting the gas feeding line, i.e. a flexible hose, to the hollow curved connector 20. The gas feeding line is fixed to the upstream end 25 of the tubular connecting piece 22 as shown in Figure 9.

The tubular connecting piece 22 can be easily attached to or detached from the hollow curved connector 20. Such fitting allows an easy and fast connection and disconnection of the mask from the gas circuit.

When the tubular connecting piece 22 is attached to the hollow curved connector 20, it can rotate with respect to said hollow curved connector 20.

For holding the tubular connecting piece 22 integral with the hollow curved connector 20, an annular inner groove 23 is arranged into the downstream end 26 of the tubular connecting piece 22, said annular inner groove 23 cooperating with peripheral lips 24 arranged on the external surface of the hollow curved connector 20.

The peripheral lips 24 are small wall expansions projecting away on the outer surface of the hollow curved connector 20. Preferably, the hollow curved connector 20 comprises at least two lips 24, for example two lips that are diametrically arranged on the outer surface of the hollow curved connector 20 (only one is visible on Figure 9).

When the lips 24 are inserted into the inner groove 23, a rotatable fixation of the tubular connecting piece 22 to the hollow curved connector 20 is ensured.

The gas is conveyed by the hollow curved connector 20 to the respiratory chamber 12 of the cushion 10, via the central passage 3 of the main body 2 of the mask 1 and the inlet orifice 11 of the cushion 10, thereby allowing pressurized gas to be introduced into the breathing chamber 12, where it can be delivered to the patient.

The hollow curved connector 20 and the tubular connecting piece 22 connected to said hollow curved connector 20 are generally made of polymer, e.g. plastic.

Actually, the hollow curved connector 20 comprises at each of its free ends, a connecting portion 21 for connecting it to the main body 2 of the mask 1, on the one hand, and to the tubular connecting piece 22 connected to a gas line, on the other hand.

In particular, one 21 of the connecting portions of the hollow curved connector 20 is sized and configured so as to be inserted and secured to the central passage 3 of the main body 2 of the mask 1 thereby ensuring a fluid continuity between them.

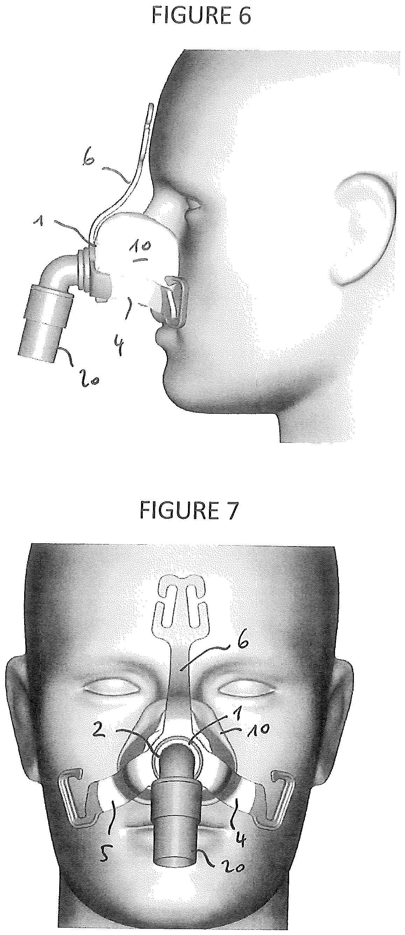

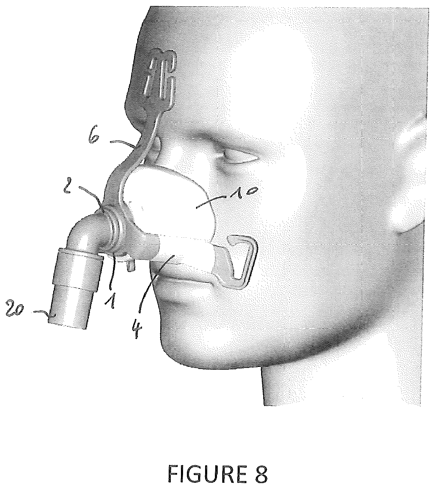

The holding arm 6 projecting upwardly (i.e. almost vertically) from the mask body 1 constitutes also a frontal support that comes into contact with the patient's forehead, whereas the two opposite lateral arms or wings 4, 5 that project laterally, i.e. on each opposite lateral sides (right and left sides) from said mask body 2, constitute right and left cheek supports, when the mask is worn by a patient, as shown in Figures 6 to 8.

Generally speaking, the respiratory mask of the present invention, such as a nasal mask, is light and comfortable to wear, thanks to the fact that is constituted of only three main sub-units or parts, namely the tubular main body 2 that has very limited dimensions (i.e. weight and sizes) and which carries the 3 projecting arms 4, 5, 6; the cushion 10 and the "L"-shape connector 20.

Such a simple architecture leads to a reduction of the overall size and weight of the mask, thereby allowing efficient positioning and securing of the mask on the patient's face, as well as a good tightness (i.e. seal) and an improved comfort of use for the patient.

The nasal respiratory mask of the present invention can be used in a method not part of the invention, for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask (1, 2, 3), preferably a nasal mask, constituted of 4 parts assembled together comprising :
- a first part made of a main body (1) having a tubular-shape portion (2) and a central passage (3), a holding arm (6) projecting upwardly from said main body (1), and two lateral arms (4, 5) projecting laterally from said main body (1), the two lateral arms (4, 5) and the holding arm (6) being integrally fixed to said tubular-shape portion (2) of the main body (1),
- a second part comprising a flexible cushion (10) fixed to a rear side (2b) of the tubular-shape portion (2) of the main body (1),
- a third part comprising a hollow curved connector (20) fixed to a front side (2a) of the tubular-shape portion (2) of the main body (1), said hollow curved connector (20) being rotatable with respect to the main body (1), and
- a fourth part comprising a tubular connecting piece (22) detachably fixed to the hollow curved connector (20), said tubular connecting piece (22) being rotatable with respect to the hollow curved connector (20),
**characterized in that**:
- the main body (1) is essentially constituted of a hollow tube element forming the tubular-shape portion (2) and the central passage (3), and
- the tubular-shape portion (2) forming the main body (1) has a generally cylindrical or tronconical shape, said tubular-shape portion (2) having a length of between 1.5 and 8 cm, and the central passage (3) having an inner diameter of between 1 and 4 cm.

2. Respiratory mask according to Claim 1, **characterized in that** the two lateral arms (4, 5) and the holding arm (6) are integral with the peripheral wall of said tubular portion (2).

3. Respiratory mask according to any one of the preceding Claims, **characterized in that** the tubular-shape portion (2) forming the main body (1), the holding arm (6) and of the two lateral arms (4, 5) are molded in one piece.

4. Respiratory mask according to any one of the preceding Claims, **characterized in that** the tubular-shape portion (2) forming the main body (1), the holding arm (6) and of the two lateral arms (4, 5) are made of a flexible material, preferably made of a polymer material.

5. Respiratory mask according to any one of the preceding Claims, **characterized in that** the two lateral arms (4, 5) and the holding arm (6) each comprises a proximal end (4b, 5b ; 6b) integral with the tubular-shape portion (2) of the main body (1), and a free distal end (4a, 5a; 6a).

6. Respiratory mask according to any one of the preceding Claims, **characterized in that** the free distal ends (6a) of the holding arm (6) and of the two lateral arms (4, 5) comprise a strap-fixing system (7, 8) for fixing thereto straps of a headgear.

7. Respiratory mask according to claim 6, **characterized in that** the strap-fixing system (7, 8) carried by the free distal ends (6a) of the holding arm (6) and of the two lateral arms (4, 5) comprise at least one slot (7) and/or a fixing hook (8).

8. Respiratory mask according to any one of the preceding Claims, **characterized in that** the flexible cushion (10) comprises a respiratory chamber (12) with an aperture (15) adapted for receiving at least part of the patient's nose, when the patient wears the mask, and an inlet orifice (11).

9. Respiratory mask according to any one of the preceding Claims, **characterized in that** at least a region (6c, 4c, 5c) of the holding arm (6) and of the two lateral arms (4, 5) is configured or shaped so as to spouse the external profile of the cushion (10).

10. Respiratory mask according to any one of the preceding Claims, **characterized in that** the tubular-shape portion (2) is traversed by the central passage (3) for conveying a gas comprising an inlet orifice at a front side (2a) and an outlet orifice at a rear side (2b) having each a diameter of between 1 and 2.5 cm.

11. Respiratory mask according to any one of the preceding Claims, **characterized in that** the hollow curved connector (20) comprises lip portions (24) cooperating with a groove (23) arranged into the tubular connecting piece (22) for ensuring a rotatable and detachable connection of the tubular connecting piece (22) to the hollow curved connector (20).

12. Respiratory mask according to any one of the preceding Claims, **characterized in that** the upstream end (25) of the tubular connecting piece (22) is configured for connecting a gas line thereto.

13. Respiratory mask according to claim 5, **characterized in that** it further comprises a headgear comprising several straps connected to the free distal ends (6a, 4a, 5a) of the holding arm (6) and of the two lateral arms (4, 5).

14. Assembly comprising a gas delivery device and a respiratory mask according to any one of the preceding Claims, preferably the gas delivery device is connected to the respiratory mask by means of a gas line, such as a flexible hose, connected to the tubular connecting piece (22) fixed to the hollow curved connector (20), said hollow curved connector (20) being fixed to the front side (2a) of the tubular portion (2) of the main body (1) of the mask.

## Patentansprüche

1. Atemmaske (1, 2, 3), vorzugsweise eine Nasenmaske, bestehend aus 4 zusammengesetzten Teilen, umfassend:
- einen ersten Teil aus einem Hauptkörper (1) mit einem röhrenförmigen Abschnitt (2) und einem zentralen Durchlass (3), einem Haltearm (6), der von dem Hauptkörper (1) nach oben vorsteht, und zwei Seitenarmen (4, 5), die seitlich von dem Hauptkörper (1) vorstehen, wobei die beiden Seitenarme (4, 5) und der Haltearm (6) einstückig an dem röhrenförmigen Abschnitt (2) des Hauptkörpers (1) befestigt sind,
- einen zweiten Teil, der ein flexibles Kissen (10) umfasst, das an einer Rückseite (2b) des röhrenförmigen Abschnitts (2) des Hauptkörpers (1) befestigt ist,
- einen dritten Teil, der einen hohlen gebogenen Stutzen (20) umfasst, der an einer Vorderseite (2a) des röhrenförmigen Abschnitts (2) des Hauptkörpers (1) befestigt ist, wobei der hohle gebogene Stutzen (20) in Bezug auf den Hauptkörper (1) drehbar ist, und
- einen vierten Teil, der ein rohrförmiges Anschlussstück (22) umfasst, das abnehmbar an dem hohlen gebogenen Stutzen (20) befestigt ist, wobei das rohrförmige Anschlussstück (22) in Bezug auf den hohlen gebogenen Stutzen (20) drehbar ist,
**dadurch gekennzeichnet, dass**:
- der Hauptkörper (1) im Wesentlichen aus einem hohlen Rohrelement besteht, das den röhrenförmigen Abschnitt (2) und den zentralen Durchlass (3) bildet, und
- der röhrenförmige Abschnitt (2), der den Hauptkörper (1) bildet, eine allgemein zylindrische oder tronkonische Form aufweist, wobei der röhrenförmige Abschnitt (2) eine Länge zwischen 1,5 und 8 cm aufweist und der zentrale Durchlass (3) einen Innendurchmesser zwischen 1 und 4 cm aufweist.

2. Atemmaske nach Anspruch 1, **dadurch gekennzeichnet, dass** die zwei Seitenarme (4, 5) und der Haltearm (6) einstückig mit der Umfangswand des röhrenförmigen Abschnitts (2) ausgebildet sind.

3. Atemmaske nach einem der vorstehenden Ansprüche, **dadurch gekennzeichnet, dass** der röhrenförmige Abschnitt (2), der den Hauptkörper (1) bildet, der Haltearm (6) und die zwei Seitenarme (4, 5) in einem Stück gegossen sind.

4. Atemmaske nach einem der vorstehenden Ansprüche, **dadurch gekennzeichnet, dass** der röhrenförmige Abschnitt (2), der den Hauptkörper (1) bildet, der Haltearm (6) und die zwei Seitenarme (4, 5) aus einem flexiblen Material hergestellt sind, vorzugsweise aus einem Polymermaterial hergestellt sind.

5. Atemmaske nach einem der vorstehenden Ansprüche, **dadurch gekennzeichnet, dass** die zwei Seitenarme (4, 5) und der Haltearm (6) jeweils ein proximales Ende (4b, 5b; 6b), einstückig mit dem röhrenförmigen Abschnitt (2) des Hauptkörpers (1), und ein freies distales Ende (4a, 5a; 6a) umfassen.

6. Atemmaske nach einem der vorstehenden Ansprüche, **dadurch gekennzeichnet, dass** die freien distalen Enden (6a) des Haltearms (6) und der zwei Seitenarme (4, 5) ein Bänderbefestigungssystem (7, 8) umfassen, um daran Bänder einer Kopfbedeckung zu befestigen.

7. Atemmaske nach Anspruch 6, **dadurch gekennzeichnet, dass** das Bänderbefestigungssystem (7, 8), das von den freien distalen Enden (6a) des Haltearms (6) und der zwei Seitenarme (4, 5) getragen wird, mindestens einen Schlitz (7) und/oder einen Befestigungshaken (8) umfasst.

8. Atemmaske nach einem der vorstehenden Ansprüche, **dadurch gekennzeichnet, dass** das flexible Kissen (10) eine Atemkammer (12) mit einer Öffnung (15), die zum Aufnehmen mindestens eines Teils der Nase des Patienten angepasst ist, wenn der Patient die Maske trägt, und eine Einlassöffnung (11) umfasst.

9. Atemmaske nach einem der vorstehenden Ansprüche, **dadurch gekennzeichnet, dass** mindestens ein Bereich (6c, 4c, 5c) des Haltearms (6) und der zwei Seitenarme (4, 5) so konfiguriert oder geformt ist, dass er sich an das äußere Profil des Kissens (10) anpasst.

10. Atemmaske nach einem der vorstehenden Ansprüche, **dadurch gekennzeichnet, dass** der röhrenförmige Abschnitt (2) von dem zentralen Durchlass (3) zum Fördern eines Gases durchquert wird, der eine Einlassöffnung an einer Vorderseite (2a) und eine Auslassöffnung an einer Rückseite (2b) umfasst, die jeweils einen Durchmesser zwischen 1 und 2,5 cm aufweisen.

11. Atemmaske nach einem der vorstehenden Ansprüche, **dadurch gekennzeichnet, dass** der hohle gebogene Stutzen (20) Lippenabschnitte (24) umfasst, die mit einer Nut (23) zusammenwirken, die in dem rohrförmigen Anschlussstück (22) angeordnet ist, um eine drehbare und abnehmbare Verbindung des rohrförmigen Anschlussstücks (22) mit dem hohlen gebogenen Stutzen (20) sicherzustellen.

12. Atemmaske nach einem der vorstehenden Ansprüche, **dadurch gekennzeichnet, dass** das stromaufwärtige Ende (25) des rohrförmigen Anschlussstücks (22) zum Anschließen einer Gasleitung daran konfiguriert ist.

13. Atemmaske nach Anspruch 5, **dadurch gekennzeichnet, dass** sie weiter eine Kopfbedeckung umfasst, die mehrere Bänder umfasst, die mit den freien distalen Enden (6a, 4a, 5a) des Haltearms (6) und der zwei Seitenarme (4, 5) verbunden sind.

14. Anordnung, umfassend eine Gasabgabevorrichtung und eine Atemmaske nach einem der vorstehenden Ansprüche, wobei die Gasabgabevorrichtung vorzugsweise über eine Gasleitung, wie einen flexiblen Schlauch, mit der Atemmaske verbunden ist, die mit dem rohrförmigen Anschlussstück (22) verbunden ist, das an dem hohlen gebogenen Stutzen (20) befestigt ist, wobei der hohle gebogene Stutzen (20) an der Vorderseite (2a) des röhrenförmigen Abschnitts (2) des Hauptkörpers (1) der Maske befestigt ist.

## Revendications

1. Masque respiratoire (1, 2, 3), de préférence un masque nasal, constitué de 4 parties assemblées ensemble comprenant :
- une première partie faite d'un corps principal (1) présentant une portion de forme tubulaire (2) et un passage central (3), une branche de maintien (6) en saillie vers le haut à partir dudit corps principal (1), et deux branches latérales (4, 5) en saillie latéralement à partir dudit corps principal (1), les deux branches latérales (4, 5) et la branche de maintien (6) étant fixées d'un seul tenant à ladite portion de forme tubulaire (2) du corps principal (1),
- une deuxième partie comprenant un coussinet souple (10) fixé à un côté arrière (2b) de la portion de forme tubulaire (2) du corps principal (1),
- une troisième partie comprenant un connecteur incurvé creux (20) fixé à un côté avant (2a) de la portion de forme tubulaire (2) du corps principal (1), ledit connecteur incurvé creux (20) étant rotatif par rapport au corps principal (1), et
- une quatrième partie comprenant une pièce de connexion tubulaire (22) fixée de façon amovible au connecteur incurvé creux (20), ladite pièce de connexion tubulaire (22) étant rotative par rapport au connecteur incurvé creux (20),
**caractérisé en ce que** :
- le corps principal (1) est sensiblement constitué d'un élément de tube creux formant la portion de forme tubulaire (2) et le passage central (3), et
- la portion de forme tubulaire (2) formant le corps principal (1) présente une forme globalement cylindrique ou tronconique, ladite portion de forme tubulaire (2) présentant une longueur entre 1,5 et 8 cm et le passage central (3) présentant un diamètre intérieur entre 1 et 4 cm.

2. Masque respiratoire selon la revendication 1, **caractérisé en ce que** les deux branches latérales (4, 5) et la branche de maintien (6) sont d'un seul tenant avec la paroi périphérique de ladite portion tubulaire (2).

3. Masque respiratoire selon l'une quelconque des revendications précédentes, **caractérisé en ce que** la portion de forme tubulaire (2) formant le corps principal (1), la branche de maintien (6) et des deux branches latérales (4, 5) sont moulées en une pièce.

4. Masque respiratoire selon l'une quelconque des revendications précédentes, **caractérisé en ce que** la portion de forme tubulaire (2) formant le corps principal (1), la branche de maintien (6) et des deux branches latérales (4, 5) sont faites d'une matière souple, de préférence faite d'une matière polymère.

5. Masque respiratoire selon l'une quelconque des revendications précédentes, **caractérisé en ce que** les deux branches latérales (4, 5) et la branche de maintien (6) comprennent chacune une extrémité proximale (4b, 5b; 6b) d'un seul tenant avec la portion de forme tubulaire (2) du corps principal (1), et une extrémité distale libre (4a, 5a ; 6a).

6. Masque respiratoire selon l'une quelconque des revendications précédentes, **caractérisé en ce que** les extrémités distales libres (6a) de la branche de maintien (6) et des deux branches latérales (4, 5) comprennent un système de fixation de courroie (7, 8) pour fixer à ce dernier les courroies d'un harnais.

7. Masque respiratoire selon la revendication 6,
**caractérisé en ce que** le système de fixation de courroie (7, 8) porté par les extrémités distales libres (6a) de la branche de maintien (6) et des deux branches latérales (4, 5) comprend au moins une fente (7) et/ou un crochet de fixation (8).

8. Masque respiratoire selon l'une quelconque des revendications précédentes, **caractérisé en ce que** le coussinet souple (10) comprend une chambre respiratoire (12) avec une ouverture (15) conçue pour recevoir au moins une partie du nez d'un patient, lorsque le patient porte le masque, et un orifice d'entrée (11).

9. Masque respiratoire selon l'une quelconque des revendications précédentes, **caractérisé en ce qu'**au moins une région (6c, 4c, 5c) de la branche de maintien (6) et des deux branches latérales (4, 5) est configurée ou mise en forme afin d'épouser le profil externe du coussinet (10).

10. Masque respiratoire selon l'une quelconque des revendications précédentes, **caractérisé en ce que** la portion de forme tubulaire (2) est traversée par le passage central (3) pour transporter un gaz comprenant un orifice d'entrée au niveau d'un côté avant (2a) et un orifice de sortie au niveau d'un côté arrière (2b) présentant chacun un diamètre entre 1 et 2,5 cm.

11. Masque respiratoire selon l'une quelconque des revendications précédentes, **caractérisé en ce que** le connecteur incurvé creux (20) comprend des portions formant lèvres (24) coopérant avec une rainure (23) agencée à l'intérieur de la pièce de connexion tubulaire (22) pour assurer une connexion rotative et amovible de la pièce de connexion tubulaire (22) au connecteur incurvé creux (20).

12. Masque respiratoire selon l'une quelconque des revendications précédentes, **caractérisé en ce que** l'extrémité amont (25) de la pièce de connexion tubulaire (22) est configurée pour connecter une conduite de gaz à cette dernière.

13. Masque respiratoire selon la revendication 5,
**caractérisé en ce qu'**il comprend en outre un harnais comprenant plusieurs courroies connectées aux extrémités distales libres (6a, 4a, 5a) de la branche de maintien (6) et des deux branches latérales (4, 5).

14. Ensemble comprenant un dispositif de délivrance de gaz et un masque respiratoire selon l'une quelconque des revendications précédentes, de préférence le dispositif de délivrance de gaz est connecté au masque respiratoire au moyen d'une conduite de gaz, comme un tuyau souple, connecté à la pièce de connexion tubulaire (22) fixée au connecteur incurvé creux (20),
ledit connecteur incurvé creux (20) étant fixé au côté avant (2a) de la portion tubulaire (2) du corps principal (1) du masque.
